# FASCICULE DE BREVET EUROPEEN

(11) **EP 2 367 624 B1**
(45) Date de publication et mention de la délivrance du brevet: **06.04.2016**
(21) Numéro de dépôt: 09805740.9
(22) Date de dépôt: 18.12.2009
(51) Int. Cl.: B01J 19/00, B01J 19/24, C07C 263/10, C07C 265/14

(54) **UTILISATION D'UN RÉACTEUR DE TYPE PISTON POUR LA MISE EN OEUVRE D'UN PROCÉDÉ DE PHOSGÉNATION**
VERWENDUNG EINES KOLBENREAKTORS ZUM UMSETZEN EINES PHOSGENIERUNGSVERFAHRENS
USE OF A PISTON REACTOR TO IMPLEMENT A PHOSGENATION PROCESS

(30) Priorité: 18.12.2008 FR 0858782
(43) Date de publication de la demande: 28.09.2011
(73) Titulaire: Vencorex France, 69800 Saint-Priest (FR)
(72) Inventeur: PERRET, Nicolas, 38080 Saint-Alban-de-Roche (FR); REVELANT, Denis, 69740 Genas (FR)
(74) Mandataire: Colombet, Alain André
(86) Numéro de dépôt international: PCT/FR2009/052605
(87) Numéro de publication internationale: WO 2010/076515

(56) Documents cités:
- FR-A- 1 469 105
- FR-A- 2 503 146
- US-A- 3 552 933
- US-A- 4 096 214

## Description

La présente invention concerne l'utilisation d'un réacteur de type piston pour la mise en oeuvre d'un procédé de phosgénation.

L'utilisation de réacteur de type piston ou tubulaire pour réaliser des procédés de phosgénation est décrite, par exemple dans le brevet FR 1 578 808.

Le procédé de phosgénation est parfois réalisé en plusieurs étapes. Ainsi, la demande US2006/0025556 décrit un procédé de phosgénation en deux étapes, la première étant de préférence réalisée sous haute température et haute pression dans un réacteur de type piston adiabatique. La demande DE 102 60 094 décrit un procédé de phosgénation sous haute température et haute pression avec détente successive du milieu réactionnel dans un réacteur et des colonnes réactives. Le brevet US 7 112 694 rapporte un procédé de phosgénation sous haute température et haute pression dans une cascade de réacteurs de type piston. La multiplication du nombre d'étapes et de réacteurs pour mettre en oeuvre la phosgénation rend le procédé de phosgénation et l'installation pour le mettre en oeuvre complexes. La sécurité des personnels est également plus difficile à assurer au vu de la complexité des installations et de la multiplication du nombre d'éléments des installations comportant du phosgène, qui est très toxique par inhalation.

Les procédés de phosgénation de l'art antérieur présentent également d'autres inconvénients : les volumes réactionnels sont généralement importants, des produits indésirables se forment lors de la réaction, qui d'une part empêchent l'écoulement des fluides par formation de résidus dans les réacteurs et les tuyaux, et d'autre part conduisent à une baisse de rendement de la réaction de phosgénation.

Les ouvrages *Turbulent mixing and chemical reactions* (Jerzy Baldyga & John R. Bourne) et *Génie de la réaction chimique conception et fonctionnement des réacteurs* (J. Villermaux) rapportent qu'un réacteur de type piston est particulièrement adapté pour mettre en oeuvre des réactions de type compétitif (à savoir lorsque qu'au moins deux réactions sont en compétition l'une par rapport à l'autre)/ consécutif (à savoir lorsqu'au moins deux réactions se suivent). Or la réaction de phosgénation d'une amine correspond exactement à ce cas de figure puisque l'isocyanate formé peut à son tour réagir sur l'amine de départ de façon compétitive pour former un sous produit non valorisable de type urée :
R-NH₂ + COCl₂ → RNH-CO-Cl + HCl ⇄ R-NCO + 2 HCl : phosgénation
R-NCO + R-NH₂ → R-NH-CO-NH-R : formation d'urée compétitive
Les ouvrages susmentionnés enseignent que le réacteur piston sans rétromélange est particulièrement adapté pour la mise en oeuvre de la réaction de phosgénation. En effet, dans un réacteur de type piston sans rétromélange, les molécules réagissant entre elles se déplacent dans une direction unique. Ainsi, le produit formé, à savoir l'isocyanate, ne revient pas dans la zone de réaction dans laquelle se trouve l'amine. Par conséquent, la réaction de formation d'urée due à la réaction entre l'isocyanate et l'amine est défavorisée.

Un des objectifs de la présente invention est de fournir un procédé de phosgénation en continu qui permette de préparer en une seule étape un (poly)isocyanate avec un bon rendement, sans formation de produits secondaires et en simplifiant l'installation pour mettre en oeuvre le procédé de manière à favoriser la sécurité.

L'invention concerne l'utilisation d'un réacteur de type piston avec recycle interne pour la mise en oeuvre d'un procédé de phosgénation d'une amine.

Dans la terminologie du génie chimique, un écoulement est dit « piston » lorsque les molécules réagissant entre elles se déplacent dans une direction unique : on peut dans ce cas poser une équivalence entre l'avancement de la réaction et la distance parcourue par les molécules depuis leur injection.

Par « piston avec recycle », on entend que le flux principal de molécules réagissant entre-elles est organisé selon un écoulement de type piston et qu'une partie de ce flux principal est renvoyé de l'aval vers l'amont, proche de la zone d'injection initiale des molécules : l'écoulement global est toujours de type piston, les concentrations initiales en molécules sont pondérées par le flux aval.

Par « recycle interne », on entend que le renvoi du flux de sortie du réacteur dans le flux d'entrée est réalisée à l'intérieur du réacteur de type piston grâce à la géométrie même du réacteur, sans utilisation de moyens mécaniques externes. La géométrie du réacteur, à savoir la différence entre le diamètre du réacteur et le diamètre des jets de réactifs, crée une zone de recirculation ou recycle interne. La géométrie du réacteur permet d'allier recycle et réaction rapide. . En particulier, un réacteur de type piston avec recycle interne est exempt de canalisation de recycle externe, également nommée boucle de recyclage. Le réacteur mis en oeuvre dans l'invention est donc un système fermé, ce qui est un avantage en termes de sécurité.

La recycle interne se différencie de la recycle externe par les éléments décrits ci-après. Un dispositif à recycle externe comporte une canalisation de recyclage externe au réacteur. Une partie du milieu réactionnel sortant du réacteur est recyclé au moyen de cette canalisation de recyclage externe. Le milieu réactionnel peut éventuellement subir des étapes de séparation avant d'être recyclé (par exemple en utilisant un séparateur de gaz). Ainsi, la demande de brevet FR 1 469 105 décrit un procédé de phosgénation en deux étapes, la première étant réalisée sous pression dans un réacteur de type piston avec recycle externe, la seconde après détente du milieu réactionnel dans une colonne réactive. De même, la demande de brevet FR 2 503 146 décrit un procédé de phosgénation en deux étapes, dans lequel l'une des étapes ou les deux peuvent être réalisées dans une canalisation de recyclage tubulaire correspondant à une recycle externe.

Par « phosgénation », on désigne la réaction du phosgène (COCl₂) sur une amine pour obtenir un (poly)isocyanate en passant intermédiairement par un chlorure de carbamyle.

Par « amine », on désigne une molécule comprenant au moins une fonction amine primaire, secondaire ou tertiaire. L'amine peut être aliphatique, cyclique ou aromatique.

Les inventeurs ont mis en évidence que l'utilisation d'un réacteur de type piston avec recycle interne est particulièrement adaptée pour mettre en oeuvre un procédé de phosgénation d'une amine. En effet, ce type de réacteur permet de minimiser le temps de réaction de la phosgénation et ainsi de minimiser la formation de produits secondaires indésirables, et d'augmenter le rendement de la réaction de phosgénation.

L'invention concerne également un procédé de préparation en continu d'un (poly)isocyanate, comprenant une étape de réaction de phosgénation d'une amine dans un réacteur de type piston avec recycle interne.

Par « préparation en continu », on entend que la préparation du (poly)isocyanate est réalisée avec injection permanente de réactifs dans le réacteur.

Par « (poly)isocyanate », on entend un isocyanate, c'est-à-dire une molécule comportant une fonction isocyanate, ou un polyisocyanate, c'est-à-dire une molécule comportant au moins deux fonctions isocyanate.

Dans un mode de réalisation, le procédé de préparation en continu d'un (poly)isocyanate selon l'invention comprend les étapes consistant à :
a) injecter une amine et du phosgène dans un réacteur de type piston avec recycle interne, et
b) effectuer la réaction de phosgénation de ladite amine dans ledit réacteur de type piston avec recycle interne.

De préférence, le phosgène est introduit en quantité surstoechiométrique (c'est-à-dire en excès) par rapport à l'amine.

Dans un mode de réalisation, dans le procédé de préparation en continu d'un (poly)isocyanate selon l'invention, la durée de la réaction de phosgénation de l'étape b) est inférieure à 200 ms, notamment inférieure à 100 ms, de préférence inférieure à 50 ms, de manière encore plus préférée inférieure à 15 ms.

Le réacteur de type piston avec recycle interne permet avantageusement de minimiser la durée de réaction de phosgénation, et ainsi de minimiser la formation de produits secondaires indésirables.

Par « durée de réaction de phosgénation », on désigne le temps nécessaire entre le moment où l'amine entre dans le réacteur (mise en contact avec le phosgène), et le moment où l'amine a été totalement transformée.

Lors de la réaction de phosgénation, l'intérieur du réacteur de type piston à recycle interne mis en oeuvre peut être décomposé en deux zones distinctes (qui ne sont pas séparées physiquement l'une de l'autre), dans lesquelles l'écoulement est différent:
- une première zone agitée avec recycle interne,
- une deuxième zone d'écoulement piston.

La première zone est une zone agitée, l'agitation provenant de la recycle interne due à la géométrie du réacteur. Les flux d'amine et de phosgène changent brusquement de direction en arrivant dans le réacteur à cause de la géométrie du réacteur, ce qui provoque la recycle interne. Dans cette première zone avec recycle interne, l'amine et le phosgène sont convertis en chlorure de carbamyle selon la réaction suivante :

R-NH₂ + COCl₂ → RNH-CO-Cl + HCl

Dans la deuxième zone, l'écoulement est de type piston et les molécules se déplacent dans une direction unique. Dans cette deuxième zone, le chlorure de carbamyle est converti en isocyanate selon la réaction suivante :

RNH-CO-Cl ⇄ R-NCO + HCl

La réaction de l'amine avec le phosgène a principalement lieu dans la première zone d'écoulement piston avec recycle interne, dans une zone très proche de l'endroit du réacteur où les réactifs amine et phosgène se rencontrent. La zone de réaction est définie par le volume dans lequel se font le mélange des réactifs et la transformation totale de l'amine. En sortie de la zone de réaction, le flux sortant est composé de phosgène en excès et de (poly)isocyanate et/ou de chlorure de carbamyle (forme hydrochlorée de l'isocyanate), le chlorure de carbamyle étant généralement majoritaire par rapport au (poly)isocyanate. La concentration en amine dans le flux sortant est quasiment nulle, car toute l'amine a été transformée lors de la réaction de phosgénation. Ce flux sortant est, pour une part, évacué à l'aval du réacteur dans la zone d'écoulement piston, et pour une autre part, renvoyé par rétromélange vers la zone de réaction grâce à la recycle interne. L'arrivée de ce flux sortant vers la zone de réaction permet ainsi d'accroître localement au niveau de la zone de réaction le débit en phosgène. L'excès stoechiométrique en phosgène par rapport à l'amine est donc localement plus élevé dans la zone de réaction qu'ailleurs dans le réacteur piston. L'augmentation du ratio stoechiométrique en phosgène favorise la conversion de l'amine et donc la réaction de phosgénation.

La réaction de phosgénation étant exothermique, la température des flux sortant de la zone de réaction est plus élevée que la température des flux entrants dans cette zone (c'est-à-dire les flux d'amine et de phosgène qui sont injectés dans le réacteur). Le flux sortant de la zone de réaction renvoyé vers la zone de réaction par le rétromélange grâce à la recycle interne permet donc d'augmenter la température de la zone de réaction, ce qui favorise la cinétique de phosgénation.

L'arrivée de ce flux de recycle supplémentaire favorise le micro-mélange des réactifs dans la zone de réaction, et favorise ainsi la réaction de phosgénation.

De plus, la diminution de la quantité de produits secondaires indésirables peut être expliquée comme suit. La concentration en produits secondaires indésirables est proportionnelle au produit de la concentration en amine et de la concentration en (poly)isocyanate. Dans la mesure où l'excès stoechiométrique en phosgène est augmenté localement dans la zone de réaction par la partie du flux sortant qui revient vers la zone de réaction grâce à la recycle interne, la concentration en amine est donc localement plus faible, et la concentration en produits secondaires également.

La diminution de la quantité de produits secondaires indésirables peut également être expliquée par le fait que la recycle soit interne. En effet, lors d'une réaction de phosgénation, la composition du milieu réactionnel recyclé n'est pas la même lorsqu'une recycle interne ou externe est mise en oeuvre.

Plus précisément, généralement, lorsqu'un système avec recycle externe est utilisé, le milieu réactionnel à la sortie du réacteur est recyclé et ré-injecté en amont du réacteur par une canalisation externe. Le milieu réactionnel en sortie du réacteur comprend, outre le phosgène en excès, majoritairement de l'isocyanate. Or, lorsque l'isocyanate est ré-injecté en amont du réacteur, il est susceptible de réagir avec l'amine entrant pour former l'urée (réaction de formation d'urée en compétition avec la formation du chlorure de carbamyle).

Au contraire, lorsqu'un système avec recycle interne est utilisé, le milieu réactionnel de la première zone d'écoulement piston avec recycle interne est renvoyé vers l'aval du réacteur. Or, ce milieu réactionnel comprend plus de chlorure de carbamyle que d'isocyanate. Le chlorure de carbamyle est une forme d'isocyanate inertée par une molécule de HCl et beaucoup moins réactive vis-à-vis de l'amine que l'isocyanate lui-même. Ainsi, la réaction secondaire de formation de l'urée est défavorisée au profit de la réaction de formation du chlorure de carbamyle (qui sera transformé en isocyanate dans la deuxième zone du réacteur).

De plus, la recycle est beaucoup plus rapide avec un réacteur à recycle interne qu'avec un réacteur à recycle externe. Or, plus la vitesse de recycle est rapide, plus la formation de produits secondaires est minimisée.

La formation de produits secondaires est donc défavorisée avec une recycle interne et les rendements en isocyanate sont donc améliorés par rapport à une recycle externe. Ainsi, un réacteur à recycle interne est plus adapté à la réaction de phosgénation, qui est de type compétitif (compétition avec la formation d'urée) et consécutive, qu'un réacteur à recycle externe.

Ainsi, le procédé selon l'invention permet d'obtenir des conversions totales en amine (c'est-à-dire que toute l'amine réagit) et des rendements en (poly)isocyanate après isolation et purification supérieurs à 90%, de préférence supérieurs à 95%.

Généralement, le réacteur de type piston avec recycle interne est exempt de parois internes et de compartiments internes. Des compartiments internes ne sont en effet pas nécessaires pour que la recycle interne ait lieu. De plus, il est préférable d'éviter que des compartiments internes soient présents dans un réacteur mis en oeuvre dans une réaction de phosgénation, qui est une réaction pour laquelle le milieu réactionnel est très corrosif (principalement à cause de la présence de phosgène et d'acide chlorhydrique), ce qui conduit à un encrassement des parois.

Dans un mode de réalisation préféré, dans le procédé de préparation en continu d'un (poly)isocyanate selon l'invention, le réacteur de type piston avec recycle interne comporte une partie tubulaire précédée d'un divergent.

Par « partie tubulaire », on désigne le tube du réacteur piston.

Par « divergent », on désigne une variation croissante de diamètre du réacteur dans la zone d'entrée des réactifs. On peut ainsi avoir une partie tubulaire de diamètre d1 suivi d'un cône et d'une deuxième partie tubulaire de diamètre d2 avec d1 < d2 ou directement une partie conique au sommet de laquelle on introduit les réactifs suivie d'une partie tubulaire de grand diamètre.

Le divergent crée ainsi la recycle interne, et donc les avantages mis en évidence ci-dessus de la recycle interne.

Dans un mode de réalisation préféré, dans l'étape a) du procédé de préparation en continu d'un (poly)isocyanate selon l'invention, l'amine et le phosgène sont injectés par un système à jets d'impact.

Par « système à jets d'impact », on désigne un système qui permet d'introduire l'amine et le phosgène sous forme de jets liquides et/ou gazeux et/ou supercritiques convergents dans le réacteur.

De manière préférée, dans l'étape a) du procédé de préparation en continu d'un (poly)isocyanate selon l'invention, le système à jets d'impact est caractérisé par un angle entre le (les) jet(s) de l'amine et le (les) jet(s) du phosgène compris de 5 à 85°, notamment de 15 à 70°, de préférence de 20 à 50°.

De tels angles sont en effet particulièrement adaptés pour minimiser le temps de réaction de phosgénation.

Selon un mode de réalisation préférentiel, le système à jets d'impact susmentionné est caractérisé par :
- une vitesse de jet d'impact d'amine compris de 20 à 80 m/s, notamment de 25 à 70 m/s, de préférence de 30 à 60 m/s, et
- une vitesse du jet d'impact du phosgène de 10 à 100 m/s, notamment de 20 à 90 m/s, de préférence de 30 à 80 m/s.

De telles vitesses sont en effet particulièrement adaptées pour minimiser le temps de réaction de phosgénation.

De préférence, le système à jets d'impact est caractérisé en ce qu'il comprend des orifices d'injection choisis parmi des trous circulaires ou à variation de courbure de type elliptiques ou en forme d'étoile de manière à augmenter le ratio périmètre mouillé / diamètre hydraulique.

Par « orifice d'injection », on désigne l'ouverture du réacteur qui permet d'y injecter l'amine ou le phosgène.

Plus la surface externe des jets de réactifs est importante, mieux se fait l'échange entre les réactifs, et plus le temps de réaction de phosgénation est minimisé. Les trous elliptiques, ou en forme d'étoile sont donc mieux adaptés que les trous circulaires, pour lesquels la surface d'échange est la plus faible.

Dans un mode de réalisation du procédé selon l'invention, la recycle interne est obtenue par injection des réactifs à travers le divergent et le rapport entre le diamètre du réacteur et du diamètre des jets est compris de 3 à 100, notamment de 10 à 80, de préférence de 20 à 60.

Par « diamètre du réacteur », on désigne le diamètre interne du tube du réacteur piston.

Par diamètre des jets, on désigne le diamètre moyen du jet de réactif (d'amine ou de phosgène). Si l'orifice d'injection est un trou circulaire, ce diamètre correspond au diamètre du trou circulaire. Pour les autres types d'orifice d'injection, le diamètre de jet correspond au diamètre moyen de l'orifice d'injection.

Dans un mode de réalisation du procédé selon l'invention, le divergent a une forme conique d'angle de 7 à 90°, notamment de 15 à 80°, de préférence de 25 à 75° et les orifices d'injection sont au sommet du divergent.

La recycle interne peut principalement être améliorée en adaptant le rapport entre le diamètre du réacteur et du diamètre des jets et/ou l'angle du divergent. Les vitesses des jets d'impact d'amine et de phosgène influent peu sur la qualité de la recycle interne.

Dans un mode de réalisation du procédé selon l'invention, le rapport entre la quantité molaire de phosgène et la quantité molaire d'amine est supérieur à 2, de préférence supérieur à 4.

Ces rapports molaires sont en effet particulièrement adaptés pour convertir la totalité de l'amine en poly(isocyanate).

Dans un mode de réalisation du procédé selon l'invention, l'amine et/ou le phosgène est pur ou est en solution dans un solvant tel que le monochlorobenzène, l'orthodichlorobenzène, ou tout solvant étant d'une part capable de solubiliser l'amine et le phosgène et d'autre part non réactif avec l'amine et le phosgène (solvant inerte). Le phosgène, lorsqu'il est utilisé pur, peut être sous forme liquide ou supercritique.

Dans un mode de réalisation du procédé selon l'invention, la température dans le réacteur de type piston est de 100 à 300°C, notamment de 120 à 250°C, de préférence de 135 à 230 °.

Dans un mode de réalisation du procédé selon l'invention, la pression dans le réacteur de type piston avec recycle interne est de 5 à 100 bar, notamment de 10 à 80 bar, de préférence de 20 à 70 bar. Ces hautes pressions sont particulièrement adaptées car elles permettent de conserver le chlorure de carbamyle sous cette forme « inertée » et d'éviter sa conversion en (poly)isocyanate dans la première zone d'écoulement piston avec recycle interne, ce qui évite la formation de produits secondaires, comme explicité ci-dessus.

Dans un mode de réalisation du procédé selon l'invention, l'isocyanate est choisi parmi le tétra-, penta-, hexa- ou octa-méthylène diisocyanate, le toluène diisocyanate, l'isophorone diisocyanate, le norbornane diisocyanate, le lysine diisocyanate, le lysine triisocyanate et le 1,5-naphtylène diisocyanate.

Dans un mode de réalisation du procédé selon l'invention, l'amine est choisie parmi la 1,4-butylène diamine, la 1,5-pentylène diamine, la 1,6-hexaméthylène diamine, la 1,8-octaméthylène diamine, la toluène diamine, l'isophorone diamine, la norbornanediamine, la lysine diamine, la lysine triamine et la 1,5-naphtylène diamine.

L'invention sera comprise plus en détail au moyen des figures et des exemples suivants.
Figure 1 : Schéma général de l'installation.
Figure 2 : Schéma du réacteur de type piston avec recycle interne et divergent.

### EXEMPLE

### Exemple 1 :

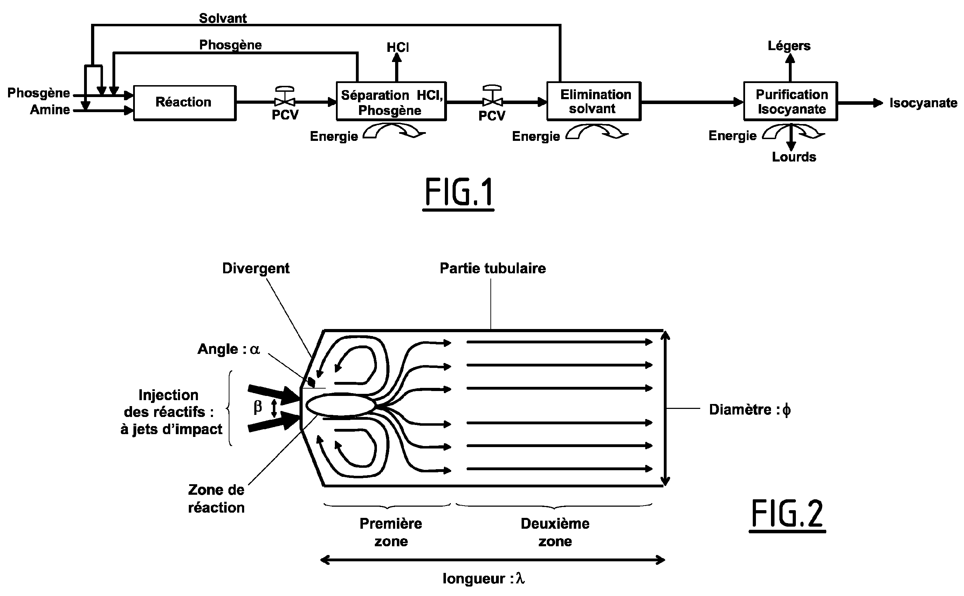

Dans un réacteur calorifugé de type piston avec recycle interne (cf figure 2) défini par une longueur λ = 2 m, un diamètre φ = 7,5 cm et un angle de divergent α = 25°, on co-introduit au moyen d'un système d'injection à jets d'impact 150 kg/h d'une solution d'hexaméthylène diamine diluée à 15% massique dans du monochlorobenzène et 250 kg/h d'une solution de phosgène dilué à 75% massique dans du monochlorobenzène. Les solutions de réactifs sont véhiculées au moyen de pompes haute pression ; la température de la solution d'hexaméthylène diamine est de 145°C et celle de la solution phosgène est de 180°C. Le système d'injection permet de contacter les jets de réactifs avec un angle β de 25° avec des vitesses de jets respectivement de 40 m/s pour la solution d'amine et de 60 m/s pour la solution de phosgène. La pression dans le réacteur est régulée à 40 bar au moyen d'une vanne de détente (PCV) et la température moyenne résultante est de 185°C. Après détente de ce milieu réactionnel, élimination de l'HCl formé et du phosgène excédentaire puis purification de l'hexaméthylène diisocyanate on obtient un rendement de transformation de 97% massique.

### Exemple 2 :

Dans un réacteur calorifugé de type piston avec recycle interne (cf figure 2) défini par une longueur λ = 3 m, un diamètre φ = 5 cm et un angle de divergent α = 20°, on co-introduit au moyen d'un système d'injection à jets d'impact 300 kg/h d'une solution de toluène diamine diluée à 18% massique dans l'ortho-dichlorobenzène et 500 kg/h d'une solution de phosgène dilué à 75% massique dans l'ortho-dichlorobenzène. Les solutions de réactifs sont véhiculées au moyen de pompes haute pression ; la température de la solution de toluène diamine est de 125°C et celle de la solution phosgène est de 100°C. Le système d'injection permet de contacter les jets de réactifs avec un angle β de 35° avec des vitesses de jets respectivement de 30 m/s pour la solution d'amine et de 40 m/s pour la solution de phosgène. La pression dans le réacteur est régulée à 35 bar au moyen d'une vanne de détente (PCV) et la température moyenne résultante est de 145°C. Après détente de ce milieu réactionnel, élimination de l'HCl formé et du phosgène excédentaire puis purification du Toluène diisocyanate on obtient un rendement de transformation de 98% massique.

## Revendications

1. Utilisation d'un réacteur de type piston avec recycle interne pour la mise en oeuvre d'un procédé de phosgénation d'une amine.

2. Procédé de préparation en continu d'un (poly)isocyanate, comprenant une étape de réaction de phosgénation d'une amine dans un réacteur de type piston avec recycle interne.

3. Procédé de préparation en continu d'un (poly)isocyanate selon la revendication 2, comprenant les étapes consistant à :
a) injecter une amine et du phosgène dans un réacteur de type piston avec recycle interne, et
b) effectuer la réaction de phosgénation de ladite amine dans ledit réacteur de type piston avec recycle interne.

4. Procédé de préparation en continu d'un (poly)isocyanate selon l'une quelconque des revendications 2 et 3, dans lequel la durée de réaction de phosgénation de l'étape b) est inférieure à 200 ms, de préférence inférieure à 15 ms.

5. Procédé de préparation en continu d'un (poly)isocyanate selon l'une quelconque des revendications 2 à 4, dans lequel le réacteur de type piston avec recycle interne comporte une partie tubulaire précédée d'un divergent.

6. Procédé de préparation en continu d'un (poly)isocyanate selon l'une quelconque des revendications 2 à 5, dans lequel, dans l'étape a), l'amine et le phosgène sont injectés par un système à jets d'impact.

7. Procédé de préparation en continu d'un (poly)isocyanate selon la revendication 6, dans lequel le système à jets d'impact est **caractérisé par** un angle entre le jet d'impact de l'amine et le jet d'impact du phosgène compris de 5 à 85° et/ou par une vitesse de jet d'impact d'amine compris de 20 à 80 m/s et une vitesse du jet d'impact du phosgène de 10 à 100 m/s.

8. Procédé de préparation en continu d'un (poly)isocyanate selon l'une quelconque des revendications 5 à 7, dans lequel la recycle interne est obtenue par injection des réactifs à travers le divergent et le rapport entre le diamètre du réacteur et du diamètre des jets est compris de 3 à 100.

9. Procédé de préparation en continu d'un (poly)isocyanate selon la revendication 8, dans lequel le divergent a une forme conique d'angle de 7 à 90° et les orifices d'injection sont au sommet du divergent.

10. Procédé de préparation en continu d'un (poly)isocyanate selon l'une quelconque des revendications 2 à 9, dans lequel la température dans le réacteur de type piston est comprise de 100 à 300°C et/ou la pression dans le réacteur de type piston avec recycle interne est comprise de 5 à 100 bar.

11. Procédé de préparation en continu d'un (poly)isocyanate selon l'une quelconque des revendications 2 à 10, dans lequel l'isocyanate est choisi parmi le tétra-, penta-, hexa- ou octa-méthylène diisocyanate, le toluène diisocyanate, l'isophorone diisocyanate, le norbornane diisocyanate, le lysine diisocyanate, le lysine triisocyanate et le 1,5-naphtylène diisocyanate.

## Patentansprüche

1. Verwendung eines Kolbenreaktors mit internem Kreislauf zur Umsetzung eines Phosgenierungsverfahrens eines Amins.

2. Verfahren zur kontinuierlichen Herstellung eines (Poly-)Isocyanats, umfassend einen Reaktionsschritt des Phosgenierens eines Amins in einem Kolbenreaktor mit internem Kreislauf.

3. Verfahren zur kontinuierlichen Herstellung eines (Poly-)Isocyanats nach Anspruch 2, umfassend die folgenden Schritte:
a) Einspritzen eines Amins und von Phosgen in einen Kolbenreaktor mit internem Kreislauf, und
b) Durchführen der Phosgenierungsreaktion des Amins in dem Kolbenreaktor mit internem Kreislauf.

4. Verfahren zur kontinuierlichen Herstellung eines (Poly-)Isocyanats nach einem der Ansprüche 2 und 3, wobei die Dauer der Phosgenie-rungsreaktion des Schritts b) weniger als 200 ms und vorzugsweise weniger als 15 ms beträgt.

5. Verfahren zur kontinuierlichen Herstellung eines (Poly-)Isocyanats nach einem der Ansprüche 2 bis 4, wobei der Kolbenreaktor mit internem Kreislauf einen rohrförmigen Abschnitt aufweist, dem ein Konus vorgeschaltet ist.

6. Verfahren zur kontinuierlichen Herstellung eines (Poly-)Isocyanats nach einem der Ansprüche 2 bis 5, wobei in Schritt a) das Amin und das Phosgen durch ein Prallstrahlsystem eingespritzt werden.

7. Verfahren zur kontinuierlichen Herstellung eines (Poly-)Isocyanats nach Anspruch 6, wobei das Prallstrahlsystem durch einen Winkel von 5 bis 85° zwischen dem Prallstrahl des Amins und dem Prallstrahl des Phosgens und/oder durch eine Geschwindigkeit des Prallstrahls des Amins von 20 bis 80 m/s und eine Geschwindigkeit des Prallstrahls des Phosgens von 10 bis 100 m/s gekennzeichnet ist.

8. Verfahren zur kontinuierlichen Herstellung eines (Poly-)Isocyanats nach einem der Ansprüche 5 bis 7, wobei der interne Kreislauf durch Einspritzen der Reagenzien durch den Konus erzielt wird und das Verhältnis zwischen dem Durchmesser des Reaktors und dem Durchmesser der Strahlen 3 bis 100 beträgt.

9. Verfahren zur kontinuierlichen Herstellung eines (Poly-)Isocyanats nach Anspruch 8, wobei der Konus eine konische Form mit einem Winkel von 7 bis 90° aufweist und sich die Einspritzöffnungen an der Spitze des Konus befinden.

10. Verfahren zur kontinuierlichen Herstellung eines (Poly-)Isocyanats nach einem der Ansprüche 2 bis 9, wobei die Temperatur in dem Kolbenreaktor 100 bis 300 °C beträgt und/oder der Druck in dem Kolbenreaktor mit internem Kreislauf 5 bis 100 bar beträgt.

11. Verfahren zur kontinuierlichen Herstellung eines (Poly-)Isocyanats nach einem der Ansprüche 2 bis 10, wobei das Isocyanat aus Tetra-, Penta-, Hexa- oder Octamethylendiisocyanat, Toluoldiisocyanat, Isophorondiisocyanat, Norbornandiisocyanat, Lysindiisocyanat, Lysintriisocyanat und 1,5-Naphthylendiisocyanat ausgewählt ist.

## Claims

1. The use of a reactor of plug-flow type with internal recycle for carrying out a process for the phosgenation of an amine.

2. A process for the continuous preparation of a (poly)isocyanate, comprising a stage of the phosgenation reaction of an amine in a reactor of plug-flow type with internal recycle.

3. The process for the continuous preparation of a (poly)isocyanate as claimed in claim 2, comprising the stages consisting in:
a) injecting an amine and phosgene into a reactor of plug-flow type with internal recycle, and
b) carrying out the phosgenation reaction of said amine in said reactor of plug-flow type with internal recycle.

4. The process for the continuous preparation of a (poly)isocyanate as claimed in either one of claims 2 and 3, in which the duration of the phosgenation reaction of stage b) is less than 200 ms, preferably less than 15 ms.

5. The process for the continuous preparation of a (poly) isocyanate as claimed in any one of claims 2 to 4, in which the reactor of plug-flow type with internal recycle comprises a tubular part preceded by a divergent.

6. The process for the continuous preparation of a (poly)isocyanate as claimed in any one of claims 2 to 5, in which, in stage a), the amine and the phosgene are injected by a system comprising impinging jets.

7. The process for the continuous preparation of a (poly)isocyanate as claimed in claim 6, in which the system comprising impinging jets is **characterized by** an angle between the impinging jet of the amine and the impinging jet of the phosgene of from 5 to 85° and/or by an amine impinging jet speed of from 20 to 80 m/s and a speed for the impinging jet of the phosgene of from 10 to 100 m/s.

8. The process for the continuous preparation of a (poly)isocyanate as claimed in any one of claims 5 to 7, in which the internal recycle is obtained by injection of the reactants through the divergent and the ratio of the diameter of the reactor to the diameter of the jets is from 3 to 100.

9. The process for the continuous preparation of a (poly)isocyanate as claimed in claim 8, in which the divergent has a conical shape with an angle of from 7 to 90° and the injection orifices are at the vertex of the divergent.

10. The process for the continuous preparation of a (poly) isocyanate as claimed in any one of claims 2 to 9, in which the temperature in the reactor of plug-flow type is from 100 to 300°C and/or the pressure in the reactor of plug-flow type with internal recycle is from 5 to 100 bar.

11. The process for the continuous preparation of a (poly)isocyanate as claimed in any one of claims 2 to 10, in which the isocyanate is chosen from tetra-, penta-, hexa- or octamethylene diisocyanate, toluene diisocyanate, isophorone diisocyanate, norbornane diisocyanate, lysine diisocyanate, lysine triisocyanate and 1,5-naphthylene diisocyanate.
